Europäisches Patentamt

European Patent Office

Office européen des brevets

(11) Publication number: **0 337 082**
**A1**

# EUROPEAN PATENT APPLICATION

(21) Application number: 89102828.4

(22) Date of filing: 18.02.89

(51) Int. Cl.⁴: **G01N 33/543 , G01N 33/74 , G01N 33/532 , G01N 33/82**

(30) Priority: 07.04.88 US 178708

(43) Date of publication of application:
18.10.89 Bulletin 89/42

(84) Designated Contracting States:
CH DE ES FR GB IT LI

(71) Applicant: **BIO-RAD LABORATORIES, INC.**
**1000 Alfred Nobel Drive**
**Hercules California 94547(US)**

(72) Inventor: **Murthy, Shashidhara H.M.**
**299 Shipley Avenue**
**Daly City California 94 015(US)**
Inventor: **Breglio, Susan E.**
**1227 Williams Street**
**Hercules, Cal. 94547(US)**
Inventor: **Phillips, Vincent E.**
**4481 Appian Way**
**El Sobrante California 94803(US)**

(74) Representative: **Patentanwälte Dipl. Ing. R.**
**Splanemann Dr. B. Reitzner Dipl. Ing. K.**
**Baronetzky**
**Tal 13**
**D-8000 München 2(DE)**

(54) Immunoassay utilizing biotin bridge with universal solid phase.

(57) An immunoassay utilizing a biotin bridge is performed with a solid phase with anti-biotin immobilized on its surface. In accordance with the assay, the solid phase is incubated with a biotinylated binding species, preferably biotinylated antibody, specific for the ligand to be detected. This is then reacted with the test sample, followed by a labeled binding member, either in a sandwich-type or competitive-type binding assay. The label is then detected in accordance with conventional detection techniques. Use of the solid phase with only a biotin-specific antibody attached thereto expands the utility and versatility of a single solid phase for use in the detection of an unlimited variety of ligands.

## IMMUNOASSAY UTILIZING BIOTIN BRIDGE WITH UNIVERSAL SOLID PHASE

This invention relates to heterogeneous immunoassays. i.e., those involving immunological bonding between species immobilized on a solid phase support and species dissolved or suspended in liquid. In particular, this invention relates to heterogeneous immunoassays utilizing biotin in a biotin/anti-biotin interaction.

## BACKGROUND AND SUMMARY OF THE INVENTION

Biospecific assays involving biotin without the use of avidin are disclosed in European Patent Application Publication No. 160900 of Abbott Laboratories, published November 13, 1985. These assays enjoy the benefit of a biotin bridge without the problems associated with avidin, which include avidin's high basicity, its destructive effect on enzyme label, and the steric hindrance problems associated with the avidin-biotin complex. The methods disclosed in this publication interpose a biotinylated antibody between the sample ligand and the tracer-bearing binding member, the latter being a labeled anti-biotin rather than a labeled antibody specific for the ligand. Both the biotinylated antibody and the tracer-bearing binding member are on the outer (exposed) side of the ligand, as opposed to the inner side which binds to an anti-ligand immobilized on a solid phase during the course of the assay.

A disadvantage of these assays is that they require solid phase with immobilized antibodies specific for the ligand of interest, and accordingly a distinctive solid phase must be prepared for each type of ligand. Furthermore, in direct immunoassays using this procedure, where an excess of solid phase binding sites are needed, the relatively high cost of preparing anti-ligand limits the number of binding sites which can be economically placed on the solid phase, and hence the range of detection of the assay.

Novel assay methods and materials have now been developed which utilize a biotin bridge, but permit the use of a common solid phase for all ligands, without loss of any of the advantages of biotin-bridge assays of the type disclosed in the Abbott published application. In accordance with this assay, a biotinylated binding member with specific binding affinity for the ligand of interest is reacted with a solid phase comprised of a solid support on whose surface anti-biotin antibody has been immobilized. The ligand is then captured by the binding member, and a labeled binding member is then introduced in a manner analogous to either the known "sandwich" techniques or the known "competitive binding" techniques, for purposes of detecting the presence and amount of ligand. By placing the biotin bridge on the solid-phase side of the ligand rather than the label side, the assay permits the use of a universal solid phase useful for all ligands regardless of their immunological binding specificity. Furthermore, the placement of the biotin bridge on the solid-phase side provides the solid phase with an increased capacity for ligand capture sites, increasing the sensitivity of the assay, broadening its range of detection, and lessening the cost of materials needed for an assay kit.

In this specification, the term "ligand" is used to denote any molecular substance for which a specific-binding material exists. Examples include antigens, antibodies, and haptens, particularly hormones and their receptor, other antigens, viruses, infectious disease agents, plant and other proteins and deoxyribonucleic acids. In certain embodiments of the invention, preferred ligands are those with at least two binding sites, notably those with two binding sites of distinct specificities different from one another.

The term "binding species" is used herein to denote any species capable of biospecific binding, particularly immunological binding with another species. Examples include antigens, antibodies, antibody fragments (either Fab or Fab'), receptors and haptens, further including modified versions and analogs of such species as well as conjugates.

The term "solid support" is intended to denote any solid material capable of immobilizing an antibody, particularly through nonspecific binding or a covalent bond. Examples are beads, test tubes, microtiter plates, nitrocellulose sheets, derivatized paper and glass, and all other known solid phase materials used in biospecific assays.

The term "detectable label" is used herein to denote a molecule, portion of a molecule, or atom whose presence can be detected distinctly from all other species in the system, by either visual observation or instrumentation techniques. Examples of such labels include those detectable by colorimetric activity, fluorometric activity and radioactivity. Examples of the labels themselves include enzyme, fluorescible species, and radioisotopes.

Further advantages and preferred embodiments will become apparent from the following description.

## DETAILED DESCRIPTION OF THE INVENTION AND PREFERRED EMBODIMENTS

The biotinylated binding species used in the present invention is a conjugate of biotin with a binding species as described above. The conjugate will be formed in such a way that the binding species retains at least a substantial amount of its binding affinity, and the biotin portion is sufficiently accessible to anti-biotin to permit immunological binding between the two. The biotinylated binding species is specific for the ligand to be detected. If the ligand is an antigen or hapten, the biotinylated binding species will be an antibody specific for the ligand. On the other hand, if the ligand is an antibody, the biotinylated binding species will either be an antigen for which the ligand is specific or a further antibody specific for the ligand antibody.

For antigen ligands which contain $\alpha$- and $\beta$-subunits or chains, the biotinylated species may be an antibody specific for either the $\alpha$-subunit or the $\beta$-subunit. For hormones having both such subunits and differing only in one of them, notably the $\beta$-subunit, a common biotinylated binding species specific for the common subunit (e.g.. the $\alpha$-subunit) may be used, with the labeled species being specific for the $\beta$-subunit and thus differing for each hormone. Conversely, the situation may be reversed: the biotinylated binding species may be specific for the $\beta$-subunit and thus differ for each hormone, with the labeled species being specific for the $\alpha$-subunit and common for all such hormones. Examples of ligands containing $\alpha$-and $\beta$-subunits are human chorionic gonadotropin (hCG), luteinizing hormone (LH), thyroid-stimulating hormone (TSH) and follicle-stimulating hormone (FSH).

In similar manner, the labeled species is a binding species bearing a detectable label, and may be antigen, antibody or hapten. The selection will depend both on the ligand and on the nature of the assay. In direct or sandwich assays, the labeled species will be one which binds in a biospecific manner to the ligand, whereas in indirect or competitive (inhibition-type) assays, the labeled species will be one which binds in a biospecific manner to the biotinylated binding species with an affinity comparable and preferably substantially equal to that of the ligand.

The assay itself may be performed in accordance with conventional assay techniques, depending on whether the sandwich or competitive-type method is used. In accordance with the sandwich method, an excess of binding sites for the ligand is provided on the solid phase and the labeled species is one which has binding specificity for the ligand itself. Thus, all ligand in the sample binds to the solid phase through the biotinylated binding species, and each molecule of ligand thus bound is further bound to the labeled binding species, thereby immobilizing an amount of label directly proportional to the amount of ligand which binds to the solid phase through the anti-biotin and the biotinylated binding species. Thus, upon separation of the solid phase from any remaining non-immobilized label, one may read the amount of label on the solid phase as being directly proportional to the amount of ligand originally present in the test sample.

In a typical competitive assay procedure (also known as an "indirect" or "inhibition-type" immunoassay), a solid phase with a limited number of binding sites is used, and the labeled species is an analog of the ligand which, together with the ligand far exceeds the number of available binding sites on the solid phase (biotinylated binding species immobilized through the anti-biotin affixed to the solid phase). Since the labeled species (i.e., the ligand analog) does not bind to the ligand from the test sample, its measurement is an indirect indication of the amount of ligand in the test sample. By virtue of the competitive binding, the relative proportions of the ligand from the test sample and its labeled analog becoming bound to the biotinylated binding species immobilized on the solid phase is equal to the relative proportions in the entire system. Thus, by using a preselected fixed amount of the labeled analog, one may determine from the proportions ultimately found in the solid phase a value representative of the amount of ligand in the test sample. Detection is achieved by separating the bound species from those remaining in solution, and determining the amount of label either residing in the solid phase or remaining in the liquid solution. In preferred procedures, the amount on the solid phase is determined. This amount is inversely proportional to the amount of ligand present in the test sample.

In the sandwich method, the biotinylated binding species and the labeled species may both be antibodies, in which case they are both specific for the ligand sought to be detected. Either monoclonal antibodies, polyclonal antibodies or both may be used. In preferred sandwich assays, the two antibodies are specific for two different epitopes or binding sites on the ligand. As an example, for assays of ligands containing alpha and beta-chains as listed above, the biotinylated antibody may be one specific for the alpha-chain, while the labeled antibody may be one specific for the beta-chain. In a particularly preferred system, the biotinylated antibody is a polyclonal antibody specific for the alpha-chain, whereas the labeled antibody is a monoclonal antibody specific for the beta-chain. The former of course will be common to all ligands containing the alpha-chain or a chain which is the substantial equivalent thereof in terms of its immunological binding specificity, and the labeled antibody is a monoclonal antibody for the beta-chain of the specific hormone which the sample is being assayed for.

The steps to carry out either of the above procedures may be performed in accordance with conventional laboratory procedures well known to those skilled in the art of immunological assays. In some cases, two or more of the steps may be performed simultaneously. For instance, the reaction between the biotinylated binding species and the test sample may be performed simultaneously with the reaction involving the labeled species, and in some cases, these two steps may be performed simultaneously with the reaction of the biotinylated binding species with the anti-biotin antibody immobilized on the solid support. Situations or procedures in which such combinations are appropriate will be readily recognized by those skilled in the art.

Detection of the label may be done according to conventional techniques, the appropriate selection depending on the type and amount of label used, in accordance with techniques well known to those skilled in the art.

The assay materials may be supplied in the form of an assay kit. Assay kits in accordance with the present invention will contain a solid phase in the form of test tubes, beads, derivatized paper, or other conventional forms, with anti-biotin antibody immobilized on the surface, plus a solution of biotinylated binding species (preferably antibody, polyclonal or monoclonal, cell surface receptors) with a specificity for the ligand to be detected, and labeled species which is either (a) an immunological binding species (preferably an antibody, polyclonal or monoclonal) with specificity toward ligand, or (b) an analog of the ligand which competes with the ligand for the limited amount of biotinylated binding species available in a given procedure. Alternatively, the solid phase may contain the biotinylated binding species already bound to it, through an anti-biotin antibody.

The following example is offered primarily for purposes of illustration, and is intended neither to limit nor define the invention in any manner.

## EXAMPLE

A sample of human serum was tested for human chorionic gonadotropin (hCG) as follows:

A 100 $\mu$L aliquot of the sample and 100 $\mu$L of biotinylated polyclonal anti-$\alpha$-hCG were introduced into a test tube coated with an excess of polyclonal anti-biotin antibody. The test tube was placed with other similar tubes on a horizontal rotator at 190 rpm. After the test tube had been on the rotator for thirty minutes, the unbound fractions were decanted and 2 mL of wash buffer were added, then decanted. The wash was then repeated and the tube was blotted dry. An iodinated tracer was then added in the form of 100 $\mu$L of $^{125}$I-monoclonal anti-$\beta$-hCG, and the test tube was returned to the rotator for an additional thirty minutes. The tube was decanted and then washed twice with wash buffer as before, then drained and blotted. The tube was then counted in accordance with conventional $^{125}$I counting techniques for one minute, with the following results:

|  | CPM | % B/TC |
|---|---|---|
| Total Counts | 198,568 | -- |
| 0 mIU/mL hCG | 291 | 0.15 |
| 10 | 2,344 | 1.18 |
| 25 | 5,263 | 2.65 |
| 100 | 21,542 | 10.85 |
| 250 | 47,164 | 23.75 |
| 500 | 81,195 | 40.89 |
| 100,000 | 138,991 | 69.99 |
| 500,000 | 118,542 | 59.70 |
| 940,000 | 112,543 | 56.67 |

The foregoing is offered primarily for purposes of illustration. It will be readily apparent to those skilled in the art that numerous variations and modifications of the various materials and procedural steps described above may be made without departing from the spirit and scope of the invention.

## Claims

1. A method for detecting a ligand in a sample, said method comprising:

(a) incubating a biotinylated binding species susceptible to immunological binding to said ligand with anti-biotin antibody immobilized on a solid support to form ligand-specific binding sites thereon;

(b) incubating said solid support with said sample;

(c) incubating said solid support with a species bearing a detectable label, said species being selected from (i) a binding species susceptible to immunological binding to said ligand and (ii) an analog of said ligand; and

(d) detecting the presence of said label in a phase selected from (i) said liquid solution and (ii) said solid support.

2. A method in accordance with claim 1 in which said biotinylated binding species and said binding species of step (c)(i) are antibodies.

3. A method in accordance in claim 1 in which step (b) comprises binding substantially all ligand in said sample to said biotinylated binding species, step (c) comprises binding substantially all ligand thus bound to a binding species susceptible to immunological binding to said ligand and bearing a detectable label, and step (d) comprises detecting the presence of said label having thus become bound to said solid support.

4. A method in accordance with claim 1 in which step (c) comprises incubating said solid support with said analog; and step (a) includes controlling the amount of ligand-specific binding sites thus formed on said solid support such that said analog and any ligand present in said sample together exceed said amount, and controlling the amount of said analog incubated with said solid support such that the amount of said analog thus becoming bound is inversely proportional to the amount of ligand present in said sample.

5. A method in accordance with claim 4 in which step (d) comprises detecting the presence of label having thus become bound to said solid support.

6. A method in accordance with claim 4 in which said ligand is human chorionic gonadotropin, said biotinylated binding species is a biotinylated antibody specific for the $\beta$-chain of human chorionic gonadotropin, and said binding species of step (c)(i) is an antibody specific for the $\alpha$-chain of human chorionic gonadotropin.

7. A method in accordance with claim 1 in which said ligand is a hormone selected from the group consisting of human chorionic gonadotropin, luteinizing hormone, thyroid-stimulating hormone and follicle-stimulating hormone, said biotinylated binding species is an antibody specific for the $\alpha$-chain of said hormone, and said binding species of step (c)(i) is an antibody specific for the $\beta$-chain of said hormone.

8. A method in accordance with claim 1 in which steps (a), (b) and (c) are performed simultaneously.

9. An assay kit for the detection of a ligand in a sample, said assay kit comprising:

(a) a solid phase comprising a solid support having immobilized on the surface thereof anti-biotin antibody:

(b) a solution of biotinylated binding species susceptible to immunological binding to said ligand;

(c) a labeled species selected from (i) a binding species susceptible to immunological binding to said ligand and (ii) an analog of said ligand with binding specificity to said biotinylated binding species substantially equivalent to that of said ligand.

10. An assay kit in accordance with claim 9 in which said biotinylated binding species and said labeled species are antibodies.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | EP-A-0 160 900 (ABBOTT LABORATORIES) <br> * Whole document * | 1-5,8 | G 01 N 33/543 <br> G 01 N 33/74 <br> G 01 N 33/532 <br> G 01 N 33/82 |
| Y | GB-A-2 181 840 (FARMOS GROUP LTD) <br> * Page 5, lines 4-6; figure 3 * | 1-5,8-10 | |
| Y | CHEMICAL ABSTRACTS, vol. 104, 1986, page 553, abstract no. 205023x, Columbus, Ohio, US; K. TANIGUCHI et al.: "Research on avidin-like compounds. Part I. Isolation of antibiotin-antibodies by means of affinity chromatography" <br> * Abstract * | 1-5,8-10 | |
| Y | CHEMICAL ABSTRACTS, vol. 107, 1987, page 475, abstract no. 113850y, Columbus, Ohio, US; I.K. MUSHAHWAR et al.: "The utilization of biotin-antibiotin interaction for the detection of antibody to hepatitis B surface antigen (anti-HBs)", & J. VIROL. METHODS 1987, 16(1-2), 45-54 | 1-8 | TECHNICAL FIELDS SEARCHED (Int. Cl.4) <br><br> G 01 N |
| Y | PATENT ABSTRACTS OF JAPAN, vol. 9, no. 276 (P-402)[1999], 2nd November 1985; & JP-A- 60 120 255 (TEIKOKU ZOKI SEIYAKU K.K.) 27-06-1985 <br> * Abstract * | 1-8 | |
| X | FR-A-2 601 455 (LABORATOIRE DES STALLERGENES) <br> * Page 5, examples; page 9, claim 1 * | 1,3-5,8 | |

The present search report has been drawn up for all claims

| Place of search | Date of completion of the search | Examiner |
|---|---|---|
| THE HAGUE | 31-07-1989 | VAN BOHEMEN C.G. |

EPO FORM 1503 03.82 (P0401)